# EUROPEAN PATENT SPECIFICATION

(11) **EP 3 451 958 B1**
(45) Date of publication and mention of the grant of the patent: **20.12.2023**
(21) Application number: 17792990.8
(22) Date of filing: 13.04.2017
(51) Int. Cl.: A61B 18/00, A61B 18/12, A61B 90/00

(54) **RECIRCULATING COOLING SYSTEMS FOR USE WITH ENERGY DELIVERY DEVICES**
RÜCKFÜHRUNGSKÜHLSYSTEM ZUR VERWENDUNG MIT ENERGIEVERSORGUNGSVORRICHTUNGEN
SYSTÈMES DE REFROIDISSEMENT À RECIRCULATION POUR UTILISATION AVEC DES DISPOSITIFS DE DISTRIBUTION D'ÉNERGIE

(30) Priority: 03.05.2016 US 201615144927
(43) Date of publication of application: 13.03.2019
(73) Proprietor: Covidien LP, Mansfield, MA 02048 (US)
(72) Inventor: DICKHANS, William J., Longmont, Colorado 80503 (US)
(74) Representative: Maschio, Antonio
(86) International application number: PCT/US2017/027321
(87) International publication number: WO 2017/192253

(56) References cited:
- WO-A1-2014/160422
- WO-A1-2014/160422
- US-A- 6 007 571
- US-A- 6 007 571
- US-A1- 2004 162 520
- US-A1- 2011 295 245
- US-B2- 7 097 657
- US-B2- 9 101 344

## Description

### BACKGROUND

### 1. Technical Field

The present disclosure relates to the use of energy delivery devices. More particularly, the present disclosure is directed to a bag configured for use with systems for cooling energy delivery devices.

### 2. Background of the Related Art

A variety of medical conditions may be treated with the delivery of energy. For example, procedures such as tissue ablation utilize energy delivery devices such as, ablation needles, ablation probes, ablation catheters, and the like, to remove unhealthy tissue including cancer cells from a patient. The energy delivery devices may operate via direct electrical discharge, electromagnetic energy, microwave energy, or other types of energy. Energy is often passed through one or more electrodes such that tissue in contact therewith is heated to an ablative temperature.

During use, energy delivery devices may require cooling to maintain a safe operating temperature and to avoid damage to the energy delivery device and/or the surround tissue. In some cases, a cooling fluid is circulated through the energy delivery device, which draws thermal energy from the energy delivery device. The fluid is then pumped out into a receptacle or to a drain. Although such systems are adequate, they may require a constant supply of fluid, which can be wasteful and inefficient. Accordingly, a need exists for a cooling system for use with a variety of energy delivery devices with improved efficiency and cost effective cooling.

US 6007571 discloses a liquid coolant supply system for supplying a liquid coolant to a thermal therapy catheter includes a sensor control unit, a liquid coolant containment unit and mounts. The sensor control unit includes a pump, a cooling device, a temperature sensor and a pressure sensor. The liquid coolant containment unit includes a sealed reservoir, a coolant-sensor interface module and a pump interface fluidly coupled to the thermal therapy catheter. The coolant-sensor interface module includes a body defining a fluid chamber, a temperature interface supported adjacent the fluid chamber within the body, and a pressure interface supported adjacent the fluid chamber within the body. The mounts removably support the sealed reservoir, pump interface, temperature interface and pressure interface of the containment unit adjacent the cooling device, the pump, the temperature sensor and the pressure sensor. The temperature interface communicates a temperature indicant of the liquid coolant circulating through the fluid chamber to the temperature sensor. The pressure interface communicates a pressure indicant of the liquid coolant circulating through the fluid chamber to the pressure sensor. The cooling device and the pump are controlled based upon the sensed temperature and pressure of the liquid coolant.

### SUMMARY OF THE INVENTION

The invention is defined in the appended independent claims. Preferred embodiments of the invention are illustrated in the dependent claims.

### SUMMARY OF THE DISCLOSURE

Like reference numerals may refer to similar or identical elements throughout the description of the figures. As is used in the art "proximal" refers to the end of the apparatus that is closer to the user and the term "distal" refers to the end of the apparatus that is farther away from the user. Further, "clinician" refers to any medical professional (e.g., doctor, surgeon, nurse, or the like) performing a medical procedure involving the use of embodiments described herein.

A kit is disclosed for use with a recirculation cooling system. The kit includes a bag having a first wall, a second wall opposite the first wall, and a side wall defining a reservoir configured to retain a fluid therein. A first port is defined through the first wall and a second port is defined through the side wall or the second wall. The bag is configured to maximize a temperature differential between a fluid proximate the first port and a fluid proximate the second port. Further, the bag is collapsible. The bag includes a divider disposed therein to divide the reservoir into a first fluid chamber and a second fluid chamber. The first chamber in fluid communication with the first port, and the second fluid chamber in fluid communication with the second port. At least a portion of the divider is a permeable membrane or a semi-permeable membrane to permit the flow of a fluid therethrough.

The kit may further include a tubing system configured to couple to the first port and the second port to permit the reservoir to be in fluid communication with a medical device. A portion of the tubing system is configured to engage a pump which is configured to draw a fluid from the reservoir through the first port, pressurize and feed a fluid through a medical device, and pump a fluid through the second port. Further, the bag and the tubing system in combination are collapsible.

The tubing system may further include a first tube and a second tube, the first tube is integrally formed with the first port, and the second tube is integrally formed with the second port.

The bag may further include a third port proximate the second port. The third port may be disposed through the second wall of the bag. The third port may include a non-vented spike.

The second port may be defined through the side wall, and the first and second ports are disposed such that a distance therebetween is maximized.

The kit may further include a temperature sensor disposed on the bag or the tubing system. The temperature sensor may be disposed on the bag proximate the first port or the second port. The temperature sensor may be disposed on the bag between the first port and the second port.

The kit may further include a fluid flow rate indicator configured to be in fluid communication with the tubing system.

At least a portion of the tubing system extends into the reservoir of the bag through at least one of the first or second ports.

A method of cooling a medical device includes filling a reservoir of a bag with a fluid via a third port of the bag, the bag including a first wall, a second wall opposite the first wall, and a side wall defining the reservoir. A first port is defined through the first wall, a second port is defined through the side wall or the second wall, and the third port is defined through the second wall. The bag is configured to maximize a temperature differential between a fluid proximate the first port and a fluid proximate the second port. The method further includes expelling a fluid from the bag through the first port for delivery to a medical device. Additionally, the method includes receiving a fluid from the medical device through the second port into the reservoir, where a temperature differential of a fluid proximate the first port and a fluid proximate the second port is maximized.

The method may further includes coupling a first tube of a tubing system to the first port of the bag, and coupling a second tube of the tubing system to the second port of the bag.

The method may further include monitoring a temperature of a fluid in the reservoir via at least one temperature sensor disposed on the tubing system or the bag.

The method may further include monitoring a flow rate of a fluid pumped from the bag via a flow indicator interposed along the tubing system between the bag and a medical device, and adjusting the flow rate based on the monitoring.

Any of the above aspects and embodiments of the present disclosure may be combined without departing from the scope of the present disclosure.

### BRIEF DESCRIPTION OF THE DRAWINGS

The above and other aspects, features, and advantages of the present disclosure will become more apparent in light of the following detailed description when taken in conjunction with the accompanying drawings in which:
FIG. 1A is a front view of one illustrative bag of a recirculating cooling system;
FIG. 1B is a front view of a bag of a recirculating cooling system in accordance with the present invention;
FIG. 2 is a front view of one illustrative cooling system for circulating a fluid through a medical device with a tubing system and a pump;
FIG. 3 is a front view of a fluid flow indicator assembly;
FIG. 4 is a flow chart of one illustrative method of use of a recirculating cooling system, in accordance with the present disclosure; and
FIG. 5 is a flow chart of method of use of a recirculating cooling system, in accordance with the present disclosure.

### DETAILED DESCRIPTION

In general, a bag for containing cooling fluid is provided for implementation into a recirculating cooling system designed to control the temperature of a medical device during use, such as, for example, electrosurgical, cauterization, or ablation devices. The bag includes a first port to which a tube is connected to thereby allow cooling fluid to be withdrawn out of the bag and into the system. A second port is formed on a side wall of the bag and is located near a top portion of the bag. Another tube is connected to the bag via the second port and returns used cooling fluid (for example, cooling fluid already circulated through to the medical device) back into the bag. The returned cooling fluid may have a higher temperature than the cooling fluid already contained in the bag. A distance between the first and second ports provides for the dissipation of heat from the higher temperature cooling fluid returning to the bag prior to being withdrawn from the first port and being re-introduced into the system.

Particular embodiments of the present disclosure are described hereinbelow with reference to the accompanying drawings. However, the disclosed embodiments are merely exemplary in nature and may be embodied in various forms. Well known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to employ the present disclosure.

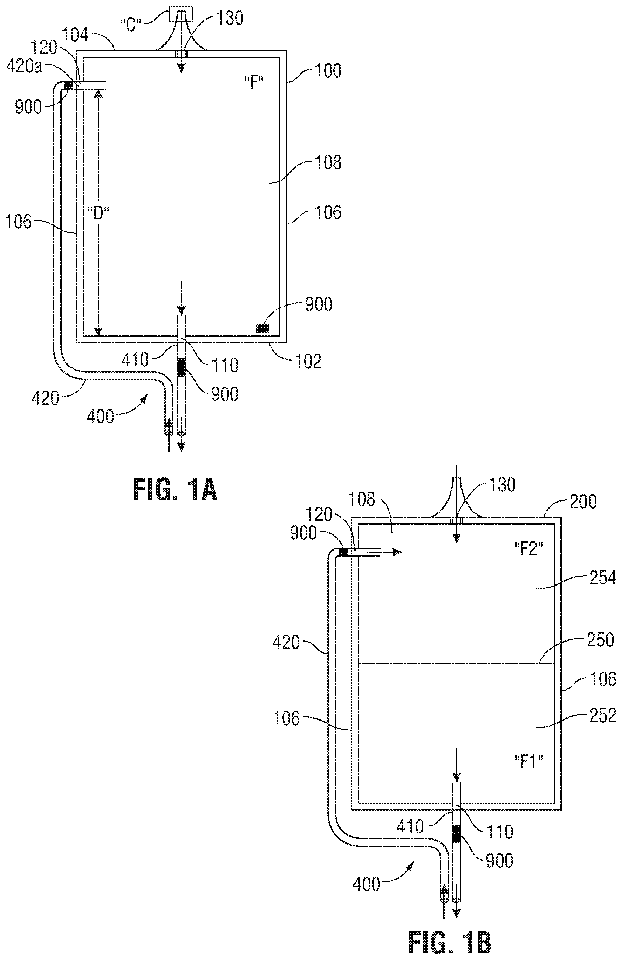

With reference to FIG. 1A, bag 100 is configured to contain a cooling fluid "F," which may be supplied to a medical device (not shown) and recirculated through cooling system 1000. Cooling fluid "F" may be a biocompatible fluid having properties to enhance thermal cooling, such as, for example, de-ionized water, sugar water, a saline solution, a dielectric solution, etc., or any combination thereof. Bag 100 may define any volumetric capacity, such as, for example, a one liter bag. It is envisioned that bag 100 may be manufactured from any suitable material, such as, for example, medical grade polymers, biocompatible polymers, etc. Further, bag 100 may include heat transfer mechanisms and/or define heat transfer properties such that, as briefly noted above, the temperature of cooling fluid "F" retained within bag 100 is reduced as thermal energy is transferred out of cooling fluid "F" into the surrounding environment. It is further envisioned that bag 100 may be wrapped or surrounded by a cooling means (not shown), such that thermal energy is expeditiously removed therefrom.

Bag 100 includes a first wall 102, a second wall 104 opposite first wall 102, and a side wall 106 therebetween. Bag 100 may define any geometric structure, e.g., rectangular, cylindrical, etc., wherein cooling fluid "F" is retained within a reservoir 108 defined by first wall 102, second wall 104, and side wall 106. Bag 100 further includes a first port 110 and a second port 120 in fluid communication with reservoir 108.

To prevent the higher temperature cooling fluid "F" from immediately returning to the medical device, first and second ports 110, 120 are not adjacent to each other. The first port 110 is defined through first wall 102 and serves as an outlet for cooling fluid "F" to feed cooling system 1000. Second port 120 is configured to receive returning fluid "F" from cooling system 1000. The second port 120 is defined through side wall 106 such that a distance "D" therebetween is maximized. In another example, first port 110 is formed through first wall 102, and second port 120 is defined through second wall 104.

No matter the particular positioning of first and/or second ports 110, 120, the distance "D" between first and second ports 110, 120 is selected to inhibit cooling fluid "F" from being expelled directly through first port 110 immediately upon return from cooling system 1000. For example, by maximizing the distance "D" which the cooling fluid "F" must travel to exit bag 100, the returning cooling fluid "F" may be retained within bag 100 for a longer duration to cool. Then, when the cooling fluid "F" is finally drawn out of reservoir 108 through first port 110 to be reintroduced into cooling system 1000, the temperature of cooling fluid "F" will be lower than it was during entry through second port 120. Further, by maximizing distance "D," a maximum temperature differential may be created between the temperature of cooling fluid "F" exiting reservoir 108 through first port 110 and a temperature of fluid "F" returning through second port 120. For example, the temperature of cooling fluid "F" exiting reservoir 108 through first port 110 may be approximately 70°F (21°C), while the temperature of cooling fluid "F" returning to reservoir 108 through second port 120 may be approximately of 140°F (60°C); hence, a temperature differential between the cooling flood "F" proximate each of first and second ports 110, 120 is approximately 70°F (39°C). It should be appreciated that first and second ports 110, 120 may be disposed on first or second walls 102, 104 or either side wall 106, such that the distance "D" therebetween is maximized.

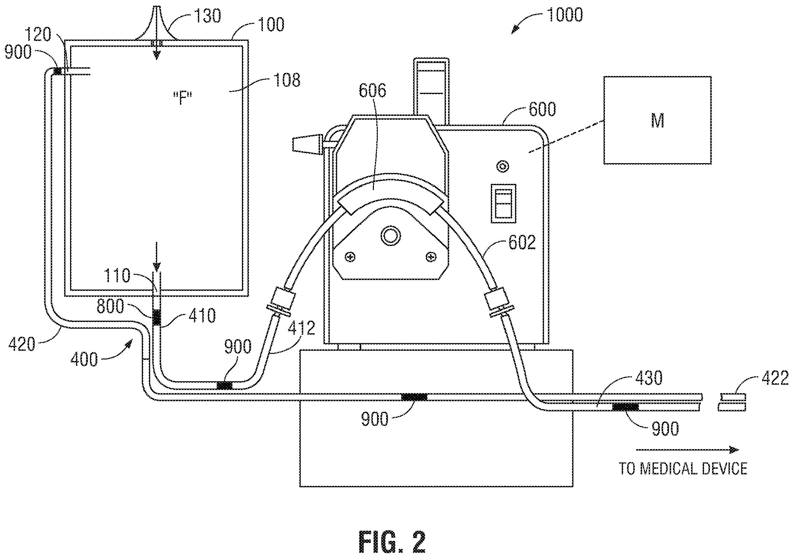

To permit cooling fluid "F" to be transferred from reservoir 108 through first port 110 into tubing system 400 and returned to reservoir 108 through second port 120, first and second ports 110, 120 are configured to engage with tubing system 400. Tubing system 400 includes a first tube 410 and a second tube 420 coupled to first port 110 and second port 120, respectively. First and second tubes 410 and 420 may be removeably coupled to the corresponding first and second ports 110, 120 via, for example, threaded engagement, luer lock or luer slip coupling, and the like. Alternatively, first and second tubes 410 and 420 may be permanently fixed, for example bonded to or integrally formed as part of bag 100. Further, one or both of first tube 410 and/or second tube 420 may be coupled to bag 100 such that a portion thereof extends into reservoir 108. A free end 412 (FIG. 2) of first tube 410 is configured to couple with an inlet port of the medical device (not shown) or alternatively may be coupled to a pump tubing 602 of a pump 600 (FIG. 2), while a free end 422 (FIG. 2) of second tube 420 is configured to couple with an outlet port of the medical device (not shown). Thus, tubing system 400 and the medical device collectively recirculate cooling fluid "F" between first port 110 and second port 120 of bag 100.

Bag 100 may further include a third port 130 in fluid communication with reservoir 108. As illustrated, third port 130 is disposed on second wall 104. In other embodiments, third port 130 may be disposed on any of first or second walls 102, 104 or side wall 106. Third port 130 may be configured to attach to a fluid source having any one of numerous conventional attachment mechanisms and thus, may include a one way valve, non-vented spike, a threaded cap, a syringe port, or any other port as is known in the art. Additionally, third port 130 may further be configured to engage a removable cap "C" (snap fit, threaded engagement) configured to enclose and secure thereto prior to, or after completion of, the filling of cooling fluid "F".

As shown in FIG. 1B, bag 200 is configured substantially the same as bag 100 of FIG. 1A, except that bag 200 in FIG. 1B further includes a divider 250. Divider 250 is attached to side wall 106 and extends across reservoir 108 to thereby divide reservoir 108 into at least two fluid chambers 252, 254. In this regard, divider 250 may be positioned in any orientation such as, for example, horizontal or diagonal, and the like. Either all or a portion of divider 250 may be a semi-permeable membrane, a permeable membrane, a non-permeable membrane, or any combination thereof. Each fluid chamber 252, 254 is in fluid communication with a corresponding one of first or second ports 110, 120, such that a distinct fluid chamber is provided for low temperature cooling fluid "F1" and higher temperature cooling fluid "F2". The exemplary illustration of FIG. 1B depicts fluid chamber 252 in fluid communication with first port 110 containing lower temperature cooling fluid "F1", while fluid chamber 254 is in fluid communication with second port 120 and is distinct and separate from fluid chamber 252 and first port 110 to receive higher temperature cooling fluid "F2". In an embodiment utilizing a permeable or semi-permeable membrane, divider 250 may be configured to regulate the rate of fluid flow therethrough, such that the transfer of the higher temperature fluid "F2" from fluid chamber 254 into fluid chamber 252 is regulated and controlled. It is envisioned that two or more dividers 250 may be utilized to define an intermediate fluid chamber or sets of fluid chambers disposed between fluid chambers 252, 254, such that a tiered configuration is created. In such an embodiment, higher temperature cooling fluid "F2" entering from second port 120 flows into fluid chamber 254, which flows into the one or more intermediate fluid chambers, wherein after a period of time fluid "F" flows into fluid chamber 252, and thus recirculates through first port 110 towards the medical device.

As noted above, bag 100 or bag 200 is implemented into recirculating cooling system 1000 to cool a medical device. For simplicity, recirculating cooling system 1000, depicted in FIG. 2, will be described including bag 100. Generally, in addition to bag 100, recirculating cooling system 1000 includes tubing system 400 and at least one pump 600. Tubing system 400 interconnects bag 100 with the medical device (not shown), which as described briefly above, has inlet and outlet ports, to crease a closed loop. Tubing system 400 may be configured to cooperatively engage any number of medical devices known in the art which utilize or require cooling along at least a portion thereof, such as, for example, an energy delivery device. Further discussion regarding medical devices configured to interact with tubing system 400, and thus recirculating cooling system 1000, may be found in commonly owned U.S. Patent No. 8,334,812, filed August 18, 2009 and entitled "Microwave Ablation Antenna Radiation Detector", U.S. Patent No. 8,430,871, filed October 28, 2009 and entitled "System and Method for Monitoring Ablation Size", U.S. Patent Application Publication No. 2014/0281961, filed March 15, 2013 and entitled "Pathway Planning System and Method", and U.S. Patent Application Publication No. 2014/0046315, filed March 15, 2013 and entitled " Microwave Ablation Catheter and Method of Utilizing the Same".

Recirculating cooling system 1000 also may include one or more pumps 600 may be used to control the pressure and flow rate of cooling fluid "F" through the system. In an embodiment, pump 600 engages first tube 410 to pressurize cooling fluid "F" within tubing system 400. Pump 600 applies pressure upon and compression to first tube 410 such that cooling fluid "F" is forced towards or away from the inlet port of the medical device. Pump 600 may be, for example, a peristaltic pump (FIG. 2), an infusion pump, a multi-channel pump, a gravity feed system, and the like. In an embodiment, pump tubing 602 may interconnect free end 412 of first tube 410 to a third tube 430, which interconnects pump tubing 602 to the inlet port of the medical device. Pump tubing 602 is configured to withstand the repetitive pressure and compressive forces encountered by the peristaltic pump during use. Hence, pump tubing 602 may be a thicker gauge and/or made of a more robust material with respect to tubing system 400. Further still, pump 600 may include a protective slip cover 606 configured to receive either first tube 410 or pump tubing 602 therethrough to provide further rigidity and structural integrity to tubing system 400, or pump tubing 602, during use. Though described herein with respect to a peristaltic pump, it should be appreciated that any pumping mechanism, as described briefly above, may be utilized in combination with tubing system 400 such that cooling fluid "F" is caused to recirculate through bag 100, tubing system 400, and the medical device.

In accordance with another embodiment of the present disclosure, recirculating cooling system 1000 may further include a fluid flow indicator connector assembly 800. Fluid flow indicator connector assembly 800 may be interconnected between bag 100 and first tube 410 or second tube 420. Alternatively or additionally, fluid flow indicator connector assembly 800 may be interposed along tubing system 400 between bag 100 and pump 600, as illustrated in FIG. 2.

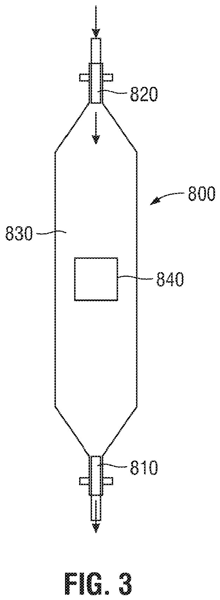

Turning now to FIG. 3, fluid flow indicator connector assembly 800 includes a first and second port 810, 820 configured to fluidly couple to bag 100 and/or tubing system 400. Fluid flow indicator connector assembly 800 includes a central portion 830 having a flow indicator 840 configured to provide auditory and/or visual indicia of properties of the flow of cooling fluid "F" therethrough, and thus through recirculating cooling system 1000. The indicia of flow indicator 840 may include, for example, bubble indicators, rotameters, Venturi devices, drip chamber indicators, float type indicators, halleffect indicators, electromagnetic indicators, or any other fluid flow or flow rate indicators known in the art. Flow indicators, such as bubble indicators and Venturi devices, may also remove any gas or vapor which may have entered recirculating cooling system 1000 and which may prevent the disruption of cooling fluid "F" therethrough. Other fluid flow indicators may also be employed to measure fluid velocity, pressure, or volumetric flow rate.

Returning now to FIG. 2, fluid flow indicator connector assembly 800 may be coupled to or include a mechanism configured to provide audible and/or visual indicia of the flow rate of cooling fluid "F" through recirculating cooling system 1000 at a remote location. In another embodiment, flow indicator 840 may be configured to provide audible and/or visual indicators if the flow of cooling fluid "F" ceases, such as, for example, a kink or blockage encountered within recirculating cooling system 1000, and/or the flow rate of cooling fluid "F" drops below a predetermined threshold.

In another embodiment of recirculating cooling system 1000, the temperature of cooling fluid "F" may be monitored at one or more points as it travels through system 1000. For example, bag 100 and/or tubing system 400 may include one or more thermocouples 900 operably connected thereto so that the temperature of cooling fluid "F" may be monitored at any location between first and second ports 110, 120 of bag 100, and through a recirculation path from first port 110 through first tube 410, through the medical device, through second tube 420 and second port 120. For example, thermocouple 900 may be placed on bag 100 proximate first port 110, second port 120, or any position therebetween; on first tube 410 or second tube 420 proximate first or second ports 110, 120, respectively, and/or proximate the inlet port and/or the outlet port of the medical device, or any position therebetween.

Thermocouple 900 may be in communication with a monitoring system "M", such as, for example, a computer including a processor and a memory which may be in communication with an energy source of the medical device and/or pump 600. Thermocouple 900 may be employed with the monitoring system "M" as a safety shut-off for pump 600 and/or the energy source of the medical device. During treatment, if the temperature of cooling fluid "F" exceeds a threshold, the energy source may be automatically shut-off to prevent undesired results to tissue and/or the medical device. The threshold may be based upon, among other things, the procedure being performed, the medical device being used, and/or the specific cooling fluid "F" being utilized. It is envisioned that flow indicator 840 may communicate with the monitoring system "M", such that the monitoring system "M" may control and adjust pump 600 and/or the energy source of the medical device dependent upon a measured rate of fluid flow of cooling fluid "F".

As briefly noted above, it is desirable to maintain the temperature of the cooling fluid "F" within a specific temperature range as it recirculates through recirculating cooling system 1000 and the medical device. The temperature of the selected cooling fluid needs to remain within a predefined range to ensure efficient cooling of the medical device and an efficient flow rate through recirculating cooling system 1000. For example, the temperature of cooling fluid "F" should be maintained below a boiling point thereof to avoid injury to the patient, damage to the medical device, and/or the introduction of air bubbles into the system, while being above a freezing temperature of the cooling fluid "F" such that the flow rate of the cooling fluid "F" is not inhibited. As will be appreciated, the configuration of first and second ports 110, 120 of bag 100 aid in the regulation of the temperature of cooling fluid "F". More particularly, the distance "D" within reservoir 108 prevents the higher temperature cooling fluid "F" from immediately returning to the medical device, and further providing for a longer duration for cooling fluid "F" to remain within reservoir 108 to facilitate cooling. For further detail of cooling systems utilizing temperature and fluid flow monitoring, reference is made to U.S. Patent No. 9,101,344, filed March 15, 2013 and entitled "Recirculating Cooling System for Energy Delivery Device".

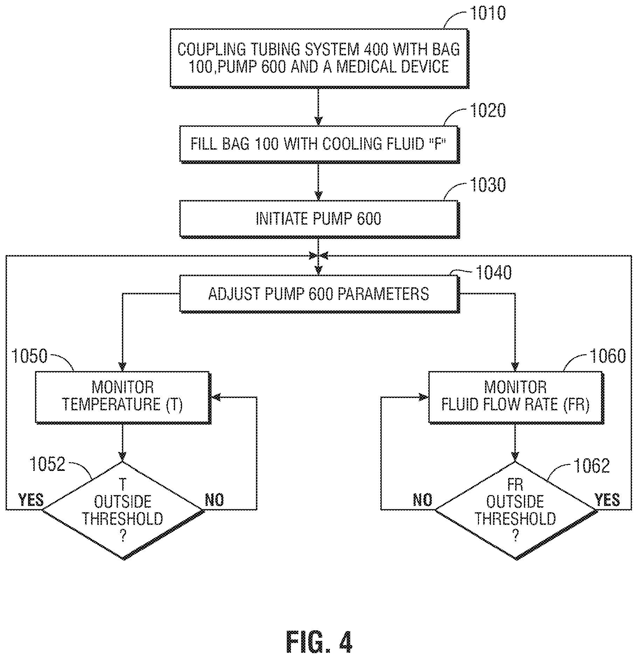

With reference to FIG. 4, a method of using bag 100 in recirculating cooling system 1000 provides for the recirculation of cooling fluid "F" through the medical device to prevent damage to the surrounding tissue and/or the medical device. In this regard, prior to the introduction of cooling fluid "F" into the medical device, a desired temperature range of cooling fluid "F" may be established where an upper limit corresponding to a temperature that is below a temperature at which tissue or the medical device is damaged, and a lower limit that is above a temperature at which the fluid flow through recirculating cooling system 1000 is inhibited. Further, a desired flow rate for cooling fluid "F" may be established where the cooling effect of cooling fluid "F" upon the medical device is achieved therebetween.

At step 1010, tubing system 400 is coupled to bag 100, pump 600, and the medical device. In an embodiment in which tubing system 400 is integrally formed with bag 100, the free ends of the tubes 410, 420 of tubing system 400 are coupled with pump 600 and the medical device, respectively. In another embodiment, tubing system 400 is coupled with pump tubing 602 with or without insertion into protective slip cover 606. At step 1020, reservoir 108 of bag 100 is filled with cooling fluid "F" through third port 130. Alternatively, bag 100 may be provided pre-filled with cooling fluid "F."

At step 1030, pump 600 is initiated to pump cooling fluid "F" from reservoir 108 to the medical device, as discussed above. It should be appreciated that pump 600 may be initiated either prior to, or during, activation of the medical device. Further, pump 600 may be run continuously or intermittently. At step 1040, the parameters of pump 600 may be adjusted to regulate the flow rate of cooling fluid "F". In one embodiment, the parameters of pump 600 are adjusted based on the temperature of cooling fluid "F", where the temperature "T" of cooling fluid "F" is monitored at step 1050 via thermocouples 900. At step 1052, if the temperature "T" of cooling fluid "F" is outside a threshold range, pump 600 is adjusted at step 1040. For example, if the temperature of cooling fluid "F" approaches the upper limit the flow rate parameter of pump 600 may be increased to increase the amount of cooling fluid "F" that is recirculated through the medical device thus increasing cooling thereof. Returning to step 1060b, if the temperature is not outside the threshold range, the method returns to step 1050.

In another embodiment, the parameters of pump 600 are adjusted based on the fluid flow rate "FR" of cooling fluid "F", where the fluid flow rate "FR" is monitored at step 1060 via fluid flow indicator connector assembly 800. At step 1062, if the fluid flow rate "FR" is outside a threshold range, pump 600 is adjusted at step 1040. For example, a drop in the fluid flow rate of cooling fluid "F" below the threshold range may indicate a blockage in recirculating cooling system 1000. It should be appreciated that the method may include both steps 1050, 1052 and 1060, 1062 either individually or in combination.

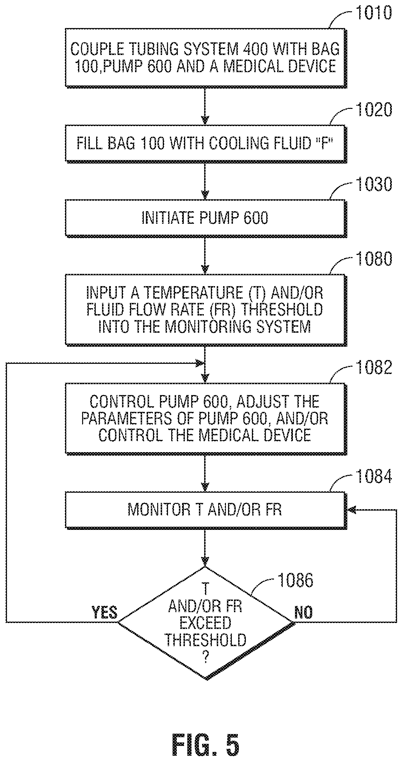

Referring to FIG. 5, a method of using bag 100 in recirculating cooling system 1000 according to another embodiment provides step 1010, where tubing system 400 is coupled to bag 100, pump 600, and the medical device. In an embodiment in which tubing system 400 is integrally formed with bag 100, the free ends of the tubes 410, 420 of tubing system 400 are coupled with pump 600 and the medical device, respectively. In another embodiment, tubing system 400 is coupled with pump tubing 602 with or without insertion into protective slip cover 606. At step 1020, reservoir 108 of bag 100 is filled with cooling fluid "F" through third port 130. Alternatively, bag 100 may be provided pre-filled with cooling fluid "F." At step 1030, pump 600 is initiated to pump cooling fluid "F" from reservoir 108 to the medical device, as discussed above. It should be appreciated that pump 600 may be initiated either prior to, or during, activation of the medical device. Further, pump 600 may be run continuously or intermittently.

At step 1080, a temperature threshold range and/or a fluid flow rate threshold range is input into the monitoring system. At step 1082, the monitoring system controls the initiation of pump 600, the adjustment of the parameters of pump 600, and/or the activation of the medical device. At step 1084, the monitoring system monitors the temperature and/or the fluid flow rate. At step 1086, a determination is made as to whether the temperature and/or fluid flow rate have exceeded the inputted threshold ranges. If the temperature and/or the fluid flow rate exceed the threshold ranges, the method returns to step 1082 where the monitoring system effects a change to the pump 600 and /or the medical device. It should be appreciated that the monitoring system may continuously or intermittently initiate pump 600, and further may provide for a safety shut-off of pump 600, and/or the medical device, if the temperature or fluid flow rate of cooling fluid "F" exceed the limits of the input threshold ranges. Otherwise, the method iterates at step 1084.

At any desired point during the procedure, pump 600 may be turned off and/or tubing system 400 may be uncoupled from the medical device, pump 600, and/or bag 100.

With reference to FIGS. 1A-3, a recirculating cooling system kit is described in accordance with the present disclosure. Kit includes one or more bags 100 and/or 200, in one or more sizes, and may be contained either individually or together in a common package. The kit may further include one or more tubing systems 400 and/or may include a plurality of tubing systems 400 having a variety of diameters. Tubing system 400 may be separate from, or unitarily formed with, bag 100, 200. Further still, bag 100, 200 and tubing system 400 may be sterilized, and/or packaged, individually or together. It is envisioned that with bag 100, 200 unitarily formed with tubing system 400, bag 100, 200 and tubing system 400 are collapsible such that a compact and low profile packaging may be provided. More particularly, the flexible and compressive nature of bag 100, 200 and tubing system 400 provides for resilient packaging, where either or both of bag 100, 200 and tubing system 400 may be folded and manipulated such that a thickness, a length, a width, or any combination thereof, may be reduced providing for a low profile compact package.

The kit may include a medical device. Further, bag 100, 200 and/or tubing system 400 may be fixed to the medical device, and thus sterilized, and packaged, concurrently therewith. The kit may further include a source of cooling fluid "F", or a variety of cooling fluids "F", such that the clinician may selectively choose an appropriate cooling fluid for the desired procedure and medical device being used. Further, the kit may include pump 600, and/or protective pump sleeve 602.

It should be appreciated that recirculating cooling system 1000 provides for bag 100, 200 to be filled with cooling fluid "F" from a variety of common receptacles, such as, for example, any standard sterile saline bags, thus eliminating the need for a specialized fluid source. Further, recirculating cooling system 1000 recirculates cooling fluid "F", thereby conserving cooling fluid "F" and eliminating the need for a fluid collection mechanism.

It should be understood that the foregoing description is only illustrative in nature. Various alternatives and modifications can be devised by those skilled in the art without departing from the disclosure.

The embodiments described with reference to the attached drawing are presented only to demonstrate certain examples of the disclosure. Other elements, steps, methods, and techniques that are insubstantially different from those described above and/or in the appended claims are also intended to be within the scope of the claimed invention.

## Claims

1. A kit for use with a recirculating cooling system, comprising:
a bag (100) including a first wall (102), a second wall (104)opposite the first wall, and a side wall (106) defining a reservoir (108) configured to retain a fluid therein, a first port (110) defined through the first wall and a second port (120),
wherein the second port is defined through the side wall or the second wall, the bag being configured to maximize a temperature differential between a fluid proximate the first port and a fluid proximate the second port by maximising a distance (D) between the first and second ports;
and wherein the bag is collapsible and further includes a divider (250) disposed therein to divide the reservoir into a first fluid chamber (252) and a second fluid chamber (254), the first chamber in fluid communication with the first port, and the second fluid chamber in fluid communication with the second port, wherein at least a portion of the divider is a permeable membrane or a semi-permeable membrane to permit the flow of a fluid therethrough.

2. The kit of claim 1, further comprising:
a tubing system (400) configured to couple to the first port and the second port to permit the reservoir to be in fluid communication with a medical device, a portion of the tubing system configured to engage a pump (600) configured to draw a fluid from the reservoir through the first port, pressurize and feed a fluid through a medical device, and pump a fluid through the second port,
wherein the bag and the tubing system in combination are collapsible.

3. The kit of claim 2 wherein the tubing system further includes a first tube (410) and a second tube (420), the first tube is integrally formed with the first port, and the second tube is integrally formed with the second port.

4. The kit of claim 2 or 3, further comprising a temperature sensor disposed on the bag or the tubing system.

5. The kit of claim 4, wherein the temperature sensor is disposed on the bag proximate the first port or the second port.

6. The kit of claim 4, wherein the temperature sensor is disposed on the bag between the first port and the second port.

7. The kit of claim 2 or 3, further comprising a fluid flow rate indicator configured to be in fluid communication with the tubing system.

8. The kit of claim 2 or 3, wherein at least a portion of the tubing system extends into the reservoir of the bag through at least one of the first or second ports.

9. The kit of any preceding claim, wherein the bag further includes a third port proximate the second port.

10. The kit of claim 8, wherein the third port is disposed through the second wall of the bag; and/or wherein the third port includes a non-vented spike.

11. The kit of any preceding claim, wherein the second port is defined through the side wall, and the first and second ports are disposed such that a distance therebetween is maximized.

12. A method of cooling a medical device, comprising:
filling a reservoir of a bag with a fluid via a third port, the bag including a first wall, a second wall opposite the first wall, and a side wall defining the reservoir, a first port defined through the first wall, a second port defined through the side wall or the second wall, and the third port defined through the second wall, the bag configured to maximize a temperature differential between a fluid proximate the first port and a fluid proximate the second port by maximising a distance (D) between the first and second ports, the bag further including a divider disposed therein to divide the reservoir into a first fluid chamber and a second fluid chamber, the first chamber in fluid communication with the first port, and the second fluid chamber in fluid communication with the second port, wherein at least a portion of the divider is a permeable membrane or a semi-permeable membrane to permit the flow of a fluid therethrough;
expelling a fluid from the bag through the first port for delivery to a medical device; and
receiving a fluid from the medical device through the second port into the reservoir, wherein a temperature differential of a fluid proximate the first port and a fluid proximate the second port is maximized.

13. The method of claim 12, further comprising:
coupling a first tube of a tubing system to the first port of the bag; and
coupling a second tube of the tubing system to the second port of the bag.

14. The method of claim 13, further comprising:
monitoring a temperature of a fluid in the reservoir via at least one temperature sensor disposed on the tubing system or the bag.

15. The method of claim 14, further comprising:
monitoring a flow rate of a fluid pumped from the bag via a flow indicator interposed along the tubing system between the bag and a medical device; and
adjusting the flow rate based on the monitoring.

## Patentansprüche

1. Satz zur Verwendung mit einem Umlaufkühlsystem, umfassend:
einen Beutel (100) mit einer ersten Wand (102), einer zweiten Wand (104) gegenüber der ersten Wand und einer Seitenwand (106), die ein Behältnis (108) definieren, das dazu ausgelegt ist, ein Fluid darin zu fassen, einer ersten Öffnung (110), die durch die erste Wand definiert ist, und einer zweiten Öffnung (120),
wobei die zweite Öffnung durch die Seitenwand oder die zweite Wand definiert ist, wobei der Beutel dazu ausgelegt ist, eine Temperaturdifferenz zwischen einem Fluid nahe der ersten Öffnung und einem Fluid nahe der zweiten Öffnung durch Maximieren eines Abstands (D) zwischen der ersten und der zweiten Öffnung zu maximieren;
und wobei der Beutel zusammenklappbar ist und ferner eine Trennvorrichtung (250) umfasst, die darin zum Teilen des Behältnisses in eine erste Fluidkammer (252) und eine zweite Fluidkammer (254) angeordnet ist, wobei die erste Kammer in Fluidverbindung mit der ersten Öffnung ist und die zweite Fluidkammer in Fluidverbindung mit der zweiten Öffnung ist, wobei zumindest ein Abschnitt der Trennvorrichtung eine durchlässige Membran oder eine halbdurchlässige Membran ist, um den Durchfluss eines Fluids dort hindurch zu ermöglichen.

2. Satz nach Anspruch 1, ferner umfassend:
ein Rohrsystem (400), das dazu ausgelegt ist, mit der ersten Öffnung und der zweiten Öffnung gekoppelt zu sein, um zu ermöglichen, dass das Behältnis in Fluidverbindung mit einer medizinischen Vorrichtung ist, wobei ein Abschnitt des Rohrsystems dazu ausgelegt ist, eine Pumpe (600) in Eingriff zu bringen, die dazu ausgelegt ist, ein Fluid aus dem Behältnis durch die erste Öffnung zu saugen, ein Fluid mit Druck zu beaufschlagen und durch eine medizinische Vorrichtung zu speisen und ein Fluid durch die zweite Öffnung zu pumpen,
wobei der Beutel und das Rohrsystem in Kombination zusammenklappbar sind.

3. Satz nach Anspruch 2, wobei das Rohrsystem ferner ein erstes Rohr (410) und ein zweites Rohr (420) umfasst, wobei das erste Rohr integral mit der ersten Öffnung ausgebildet ist und das zweite Rohr integral mit der zweiten Öffnung ausgebildet ist.

4. Satz nach Anspruch 2 oder 3, ferner umfassend einen Temperatursensor, der an dem Beutel oder dem Rohrsystem angeordnet ist.

5. Satz nach Anspruch 4, wobei der Temperatursensor an dem Beutel nahe der ersten Öffnung oder der zweiten Öffnung angeordnet ist.

6. Satz nach Anspruch 4, wobei der Temperatursensor an dem Beutel zwischen der ersten Öffnung und der zweiten Öffnung angeordnet ist.

7. Satz nach Anspruch 2 oder 3, ferner umfassend eine Fluiddurchflussratenanzeige, die dazu ausgelegt ist, in Fluidverbindung mit dem Rohrsystem zu sein.

8. Satz nach Anspruch 2 oder 3, wobei sich zumindest ein Abschnitt des Rohrsystems durch mindestens eine der ersten oder zweiten Öffnung in das Behältnis des Beutels erstreckt.

9. Satz nach einem der vorhergehenden Ansprüche, wobei der Beutel ferner eine dritte Öffnung nahe der zweiten Öffnung umfasst.

10. Satz nach Anspruch 8, wobei die dritte Öffnung durch die zweite Wand des Beutels angeordnet ist; und/oder wobei die dritte Öffnung einen unbelüfteten Dorn umfasst.

11. Satz nach einem der vorhergehenden Ansprüche, wobei die zweite Öffnung durch die Seitenwand definiert ist und die erste und zweite Öffnung derart angeordnet sind, dass ein Abstand dazwischen maximiert ist.

12. Verfahren zum Kühlen einer medizinischen Vorrichtung, umfassend:
Füllen eines Behältnisses eines Beutels mit einem Fluid über eine dritte Öffnung, wobei der Beutel eine erste Wand, eine zweite Wand gegenüber der ersten Wand und eine Seitenwand umfasst, die das Behältnis definieren, wobei eine erste Öffnung durch die erste Wand definiert ist, eine zweite Öffnung durch die Seitenwand oder die zweite Wand definiert ist und die dritte Öffnung durch die zweite Wand definiert ist, wobei der Beutel dazu ausgelegt ist, eine Temperaturdifferenz zwischen einem Fluid nahe der ersten Öffnung und einem Fluid nahe der zweiten Öffnung durch Maximieren eines Abstands (D) zwischen der ersten und zweiten Öffnung zu maximieren, wobei der Beutel ferner eine Trennvorrichtung umfasst, die darin zum Teilen des Behältnisses in eine erste Fluidkammer und eine zweite Fluidkammer angeordnet ist, wobei die erste Kammer in Fluidverbindung mit der ersten Öffnung ist und die zweite Fluidkammer in Fluidverbindung mit der zweiten Öffnung ist, wobei zumindest ein Abschnitt der Trennvorrichtung eine durchlässige Membran oder eine halbdurchlässige Membran ist, um den Durchfluss eines Fluids dort hindurch zu ermöglichen;
Ausstoßen eines Fluids aus dem Beutel durch die erste Öffnung zur Bereitstellung zu einer medizinischen Vorrichtung; und
Aufnehmen eines Fluids aus der medizinischen Vorrichtung durch die zweite Öffnung in das Behältnis, wobei eine Temperaturdifferenz eines Fluids nahe der ersten Öffnung und eines Fluids nahe der zweiten Öffnung maximiert wird.

13. Verfahren nach Anspruch 12, ferner umfassend:
Koppeln eines ersten Rohrs eines Rohrsystems mit der ersten Öffnung des Beutels; und
Koppeln eines zweiten Rohrs des Rohrsystems mit der zweiten Öffnung des Beutels.

14. Verfahren nach Anspruch 13, ferner umfassend:
Überwachen einer Temperatur eines Fluids in dem Behältnis über mindestens einen Temperatursensor, der an dem Rohrsystem oder dem Beutel angeordnet ist.

15. Verfahren nach Anspruch 14, ferner umfassend:
Überwachen einer Durchflussrate eines aus dem Beutel gepumpten Fluids über eine Durchflussanzeige, die entlang des Rohrsystems zwischen dem Beutel und einer medizinischen Vorrichtung angeordnet ist; und
Anpassen der Durchflussrate basierend auf dem Überwachen.

## Revendications

1. Kit destiné à être utilisé avec un système de refroidissement à recirculation, comprenant :
un sac (100) comprenant une première paroi (102), une deuxième paroi (104) opposée à la première paroi, et une paroi latérale (106) définissant un réservoir (108) configuré pour retenir un fluide à l'intérieur, un premier orifice (110) défini à travers la première paroi et un deuxième orifice (120),
le deuxième orifice étant défini à travers la paroi latérale ou la deuxième paroi, le sac étant configuré pour maximiser un différentiel de température entre un fluide proche du premier orifice et un fluide proche du deuxième orifice en maximisant une distance (D) entre les premier et deuxième orifices ;
et le sac étant pliable et comprenant en outre une cloison (250) disposée à l'intérieur pour diviser le réservoir en une première chambre de fluide (252) et une deuxième chambre de fluide (254), la première chambre en communication fluidique avec le premier orifice, et la deuxième chambre de fluide en communication fluidique avec le deuxième orifice, au moins une partie de la cloison étant une membrane perméable ou une membrane semi-perméable pour permettre l'écoulement d'un fluide à travers elle.

2. Kit selon la revendication 1, comprenant en outre :
un système de tubulure (400) configuré pour se coupler au premier orifice et au deuxième orifice afin de permettre au réservoir d'être en communication fluidique avec un dispositif médical, une partie du système de tubulure étant configurée pour venir en prise avec une pompe (600) configurée pour aspirer un fluide du réservoir à travers le premier orifice, mettre sous pression et alimenter par un fluide un dispositif médical, et pomper un fluide à travers le deuxième orifice,
le sac et le système de tubulure étant pliables de manière combinée.

3. Kit selon la revendication 2, le système de tubulure comprenant en outre un premier tube (410) et un deuxième tube (420), le premier tube étant formé d'un seul tenant avec le premier orifice, et le deuxième tube étant formé d'un seul tenant avec le deuxième orifice.

4. Kit selon la revendication 2 ou 3, comprenant en outre un capteur de température disposé sur le sac ou le système de tubulure.

5. Kit selon la revendication 4, le capteur de température étant disposé sur le sac à proximité du premier orifice ou du deuxième orifice.

6. Kit selon la revendication 4, le capteur de température étant disposé sur le sac entre le premier et le deuxième orifice.

7. Kit selon la revendication 2 ou 3, comprenant en outre un indicateur de débit de fluide configuré pour être en communication fluidique avec le système de tubulure.

8. Kit selon la revendication 2 ou 3, au moins une partie du système de tubulure s'étendant dans le réservoir du sac à travers au moins l'un des premier ou deuxième orifices.

9. Kit selon n'importe quelle revendication précédente, le sac comprenant en outre un troisième orifice à proximité du deuxième orifice.

10. Kit selon la revendication 8, le troisième orifice étant disposé à travers la deuxième paroi du sac ; et/ou le troisième orifice comprenant une pointe non ventilée.

11. Kit selon n'importe quelle revendication précédente, le deuxième orifice étant défini à travers la paroi latérale, et les premier et deuxième orifices étant disposés de manière à maximiser la distance entre eux.

12. Procédé de refroidissement d'un dispositif médical, comprenant :
le remplissage d'un réservoir d'un sac avec un fluide via un troisième orifice, le sac comprenant une première paroi, une deuxième paroi opposée à la première paroi, et une paroi latérale définissant le réservoir, un premier orifice défini à travers la première paroi, un deuxième orifice défini à travers la paroi latérale ou la deuxième paroi, et le troisième orifice défini à travers la deuxième paroi, le sac étant configuré pour maximiser un différentiel de température entre un fluide proche du premier orifice et un fluide proche du deuxième orifice en maximisant une distance (D) entre les premier et deuxième orifices, le sac comprenant en outre une cloison disposée à l'intérieur pour diviser le réservoir en une première chambre de fluide et une deuxième chambre de fluide, la première chambre en communication fluidique avec le premier orifice, et la deuxième chambre de fluide en communication fluidique avec le deuxième orifice, au moins une partie de la cloison étant une membrane perméable ou une membrane semi-perméable pour permettre l'écoulement d'un fluide à travers elle ;
l'expulsion d'un fluide du sac à travers le premier orifice pour le distribuer à un dispositif médical ; et
la réception d'un fluide provenant du dispositif médical à travers le deuxième orifice dans le réservoir, un différentiel de température d'un fluide proche du premier orifice et d'un fluide proche du deuxième orifice étant maximisé.

13. Procédé selon la revendication 12, comprenant en outre :
l'accouplement d'un premier tube d'un système de tubulure au premier orifice du sac ; et
l'accouplement d'un deuxième tube du système de tubulure au deuxième orifice du sac.

14. Procédé selon la revendication 13, comprenant en outre :
la surveillance de la température d'un fluide dans le réservoir par l'intermédiaire d'au moins un capteur de température disposé sur le système de tubulure ou le sac.

15. Procédé selon la revendication 14, comprenant en outre :
la surveillance du débit d'un fluide pompé à partir du sac par l'intermédiaire d'un indicateur de débit interposé le long du système de tubulure entre le sac et un dispositif médical ; et
l'ajustement du débit sur la base de la surveillance.
